# EUROPEAN PATENT APPLICATION

(11) **EP 2 977 030 A1**
(43) Date of publication of application: **27.01.2016**
(21) Application number: 14769123.2
(22) Date of filing: 31.01.2014
(51) Int. Cl.: A61F 2/915

(54) **STENT**

(30) Priority: 18.03.2013 JP 2013054568
(71) Applicant: Piolax Medical Devices, Inc., Yokohama-shi Kanagawa 240-0023 (JP)
(72) Inventor: KITANO, Masayuki, Osakasayama-shi Osaka 589-0023 (JP); IMAI, Hajime, Osakasayama-shi Osaka 589-0005 (JP); TOYOKAWA, Yoshihide, Yokohama-shi Kanagawa 240-0023 (JP); SHIRAKAWA, Kyosuke, Yokohama-shi Kanagawa 240-0023 (JP)
(74) Representative: Grünecker Patent- und Rechtsanwälte PartG mbB
(86) International application number: PCT/JP2014/052214
(87) International publication number: WO 2014/148122

(57) **Abstract**

Provided is a stent, in which a silicone film can be formed in a small thickness and uniformly and the diameter of the stent can be reduced. The stent (10) comprises: a tubular stent main body (20) which is formed by processing a metal tube or a metal sheet or weaving a metal wire material and has mesh-like openings (21); an inside cover (30) which is so coated around the stent main body (20) as to cover the mesh-like openings (21) and is composed of a polyurethane film; and an outside cover (40) which is coated on the outer periphery of the stent main body (20) and the inside cover (30) and is composed of a silicone film.

## Description

### Technical Field

The present invention relates to a stent covered with a cover, and which is to be indwelled in a tubular organ such as the bile duct, the ureter, the trachea, or the blood vessel, or another body tissue.

### Background Art

Conventionally, a stent in which mesh-like openings are covered by a cover has been widely used. Such a stent is used in applications, for example, a case where it is indwelled in a stenosed or obstructed portion in a tubular organ such as the bile duct, the ureter, the trachea, or the blood vessel to expand the tubular organ, thereby allowing the bile, the blood, or the like to easily flow, or that where it is indwelled in a portion where an aneurysm occurs, to prevent rupture of the aneurysm from occurring.

To indwell such a stent, a guide wire may be caused to reach a predetermined position of a tubular organ through the skin, by way of an endoscope, or the like, a catheter accommodating a stent in a contracted state may be transferred to a target position while being guided by the guide wire, and then the stent may be pushed out from the catheter to be indwelled.

In the case where a stent is to be indwelled in the bile duct, for example, a guide wire is passed through the papilla to be inserted to a predetermined position of the bile duct, a catheter accommodating a stent in a contracted state is inserted to a target position of the bile duct while being guided by the guide wire, and the stent is pushed out from the catheter to be indwelled in the bile duct.

Even when it is attempted to insert a guide wire and a catheter through the papilla, the catheter, the guide wire, and the like cannot be moved close to the papilla portion depending on circumstances such as existence of stenosis in the duodenum or the like. Instead, an endoscopic ultrasound (EUS) may be performed. In this case, first, the stomach and the duodenum are punctured with a puncture needle via the stomach and the duodenum by way of the endoscopic ultrasound, the bile duct is then punctured via the abdominal cavity, a guide wire is inserted into the bile duct through the puncture needle, and thereafter the puncture needle is pulled out. Then, the diameter of the puncture hole formed by the puncture needle is gradually increased by repeating an operation of using a larger tube or dilator plural times. After the puncture hole has been enlarged to a size which allows the catheter to be inserted, the catheter accommodating a stent is transferred into the bile duct, and the stent is indwelled.

As the material of the cover of the above-described stent, polyurethane, nylon, or the like is used. These materials are susceptible to hydrolysis. Therefore, the cover of a stent which is to be indwelled for a long time period in the bile duct through which the bile fluxes, the blood vessel through the blood flows, or the like is often formed by silicone which is hardly hydrolyzed.

For example, Patent Literature 1 describes a stent in which plural interstices are formed by a first thread element and a second thread element, and a matrix for closing the interstices is formed by a polymeric material made of silicone rubber.

### Citation List

### Patent Literature

**Patent Literature 1:** JP-2735389-B

### Summary of Invention

### Technical Problem

In a stent which is to be indwelled in a thin tubular organ such as the bile duct, preferably, the outer diameter is small because of the following reason. When the outer diameter of a stent ls large, the inner and outer diameters of a catheter which accommodates it are also large. In the case where the stent is indwelled by way of the endoscopic ultrasound as described above, the number of operations of pushing in and pulling out tubes or dilators is increased, and the bile often leaks from the bile duct. This causes the abdominal cavity inflammation or the like.

In order to diametrically miniaturize a stent, a silicone film may be formed to be thin with respect to the stent. However, it is not easy to form a silicone film uniformly and while preventing pinholes from being produced. It is required to form a silicone film so as to be relatively thick. Therefore, it is difficult to diametrically miniaturize a stent.

It is an object of the invention to provide a stent in which a silicone film can be formed thinly and uniformly, and the stent can be diametrically miniaturized.

### Solution to Problem

In order to attain the object, the invention provides
a stent including:
a tubular stent main body which is formed by processing a metal tube or a metal plate, or by braiding a metal wire material, and which has mesh-like openings;
an inner cover which is made of a polyurethane film, and which covers the stent main body so as to close the mesh-like openings; and
an outer cover which is made of a silicone film, and which covers an outer circumference of the stent main body and the inner cover.

The invention may provide the stent,
wherein a film thickness of the inner cover is 10 to 60 µm, a film thickness of the outer cover is 5 to 60 µm, and a total film thickness of these covers is 15 to 120 µm.

The invention may provide the stent,
wherein the stent is configured to have an outer diameter in a contracted state of 1.5 to 2.5 mm.

### Advantageous Effects of Invention

According to the invention, the outer circumference of the inner cover which is made of a polyurethane film is covered by the outer cover which is made of a silicone film having high affinity for polyurethane. Therefore, while pinholes are easily formed in the silicone film if it is used as a single layer and the thickness thereof is hardly reduced, the silicone flm can be formed thinly and uniformly, and the stent can be diametrically miniaturized. Consequently, the stent can be accommodated even in a catheter of a small inner diameter, the number of uses of tubes, dilators, or the like for increasing the diameter of a hole through which the catheter is to be passed can be reduced, and a hole which is to be formed in the bile duct or the like can be made small, thereby suppressing invasion to the body. In the case where a hole is formed in the bile duct, the amount of the bile which leaks from the bile duct to the abdominal cavity can be reduced, and therefore peritonitis can be suppressed.

The outer cover made of a silicone film which is hardly hydrolyzed can prevent the polyurethane film of the inner cover from being hydrolyzed. Therefore, the stent can be indwelled for a long time period.

Moreover, since the inner cover is made of a polyurethane film, irregularities of the inner circumference of the inner cover can be reduced, and the inner circumference can be made relatively smooth. When the stent is indwelled in a tubular organ such as the bile duct, for example, it is possible to suppress sludge such as biliary sludge from being produced inside the stent.

### Brief Description of Drawings

Figs. 1A to 1C show a stent according to an embodiment of the invention. Fig. 1A is a perspective view of a stent main body constituting the stent. Fig. 1B is a perspective view of the stent main body and an inner cover which covers the inner circumference thereof. Fig. 1C is a perspective view of a state where a part of the stent of the invention which is further covered by an outer cover is cutaway.
Fig. 2 is a sectional view of the stent.
Figs. 3A and 3B show the stent main body constituting the stent. Fig. 3A is a development view thereof. Fig. 3B is a development view of a stent main body of another example.
Fig. 4 is a diagram showing a use state of the stent.

### Description of Embodiments

Hereinafter, a stent of according to an embodiment of the invention will be described with reference to the drawings.

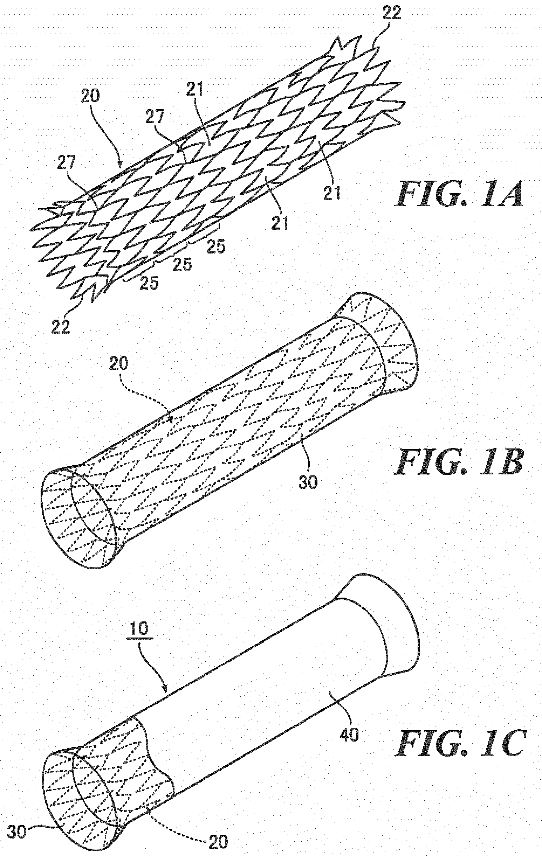

As shown in Figs. 1A to 1C, a stent 10 of the embodiment has: a cylindrical stent main body 20 which has plural mesh-like openings 21; an inner cover 30 which is made of a polyurethane film and which covers the stent main body 20 so as to close the mesh-like openings 21 of the stent main body 20; and an outer cover 40 which is made of a silicone film and which covers the outer circumference of the stent main body 20 and the inner cover 30.

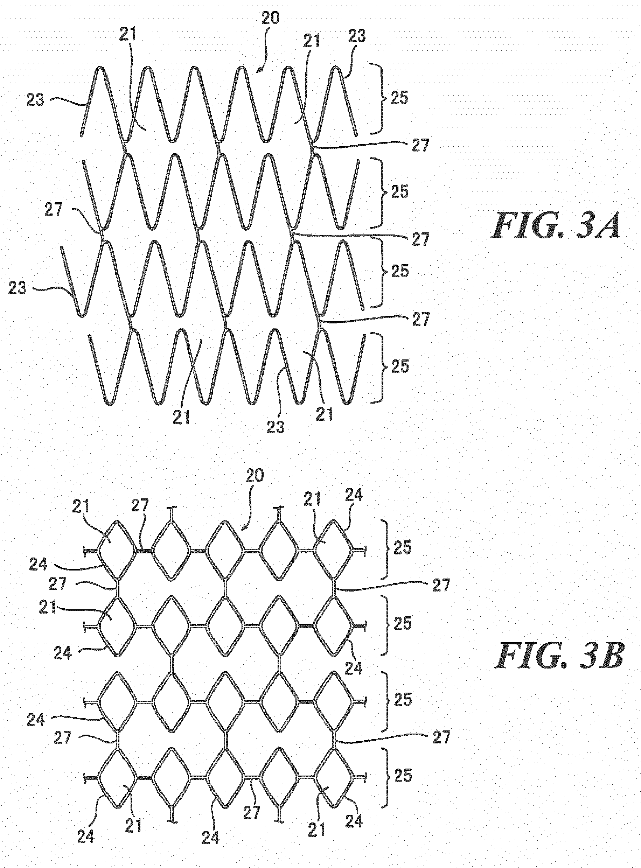

In the embodiment, the cylindrical stent main body 20 having the plural mesh-like openings 21 is formed by processing a metal cylinder (see Fig. 1A). This will be described with reference also to the development view of Fig. 3A. A metal cylinder is processed by laser processing, etching, or the like, so as to be bent into a corrugated shape and to circumferentially extend in a zig-zag manner. A circumferential unit 25 is formed by circumferentially connecting the both ends of zig-zag portions 23. The generally-cylindrical-shaped stent main body 20 is configured by axially connecting the bent portions of the zig-zag portions 23 of the circumferential units 25 through connecting portions 27.

Alternatively, as shown in Fig. 3B, the cylindrical stent main body 20 may be configured by processing a metal cylinder to form plural frame-like members 24 having openings 21, by circumferentially connecting the frame-like members 24 to form the circumferential unit 25, and by axially connecting the circumferential units 25 through plural connecting portions 27.

Alternatively, the cylindrical stent main body 20 may be configured by processing a metal plate to form plural circumferential units 25 having zig-zag portions 23 or frame-like members 24 and cylindrically bending the processed metal plate, or by braiding, joining, or tangling a metal wire material.

The above-described stent main body 20 is of the self-expandable type which is normally in the expanded state. However, the stent main body may be of the balloon expandable type in which the stent main body is previously attached to a balloon catheter or the like, and the stent main body is expanded by inflating a balloon placed thereinside. The placement pattern of the mesh-like openings 21 of the stent main body 20 is not limited to the patterns shown in Figs. 3A and 3B, and not particularly limited as long as the stent main body is structurally contractible and expandable in diameter.

In the embodiment, as shown in Fig. 1A, end portions 22, 22 of the stent main body 20 are gradually expanded in a flare-like manner toward the axially outer side of the stent main body 20. In the embodiment, the end portions 22, 22 of the stent main body 20 have a flared shape. Alternatively, one end portion 22 may be expanded in a flare-like manner while the other end portion 22 may be formed into a straight tube-like shape, or the end portions 22, 22 may be formed into a straight tube-like shape. The shape of the stent main body 20 is not limited to a cylindrical shape. For example, the whole stent main body 20 may be gradually contracted in a tapered manner from the one axial end toward the other axial end. Alternatively, the stent main body 20 may have a bifurcated portion.

The material of the stent main body 20 is not particularly limited, but a metal such as stainless steel, Ta, Ti, Pt, Au, or W, or a shape memory alloy such as a Ni-Ti alloy, a Co-Cr alloy, a Co-Cr-Ni alloy, a Cu-Zn-X (X = Al, Fe, or the like) alloy, a

Ni-Ti-X (X = Fe, Cu, V, Co, or the like) alloy may be preferably used.

At a predetermined position of the stent main body 20, an X-ray marker having an X-ray opacity may be provided. The X-ray marker may be made of Pt, Ti, Pd, Rh, Au, W, Ag, Bi, or Ta, an alloy of these metals, a synthetic resin containing powder of BaSO₄, Bi, W, or the like, or stainless steel,.

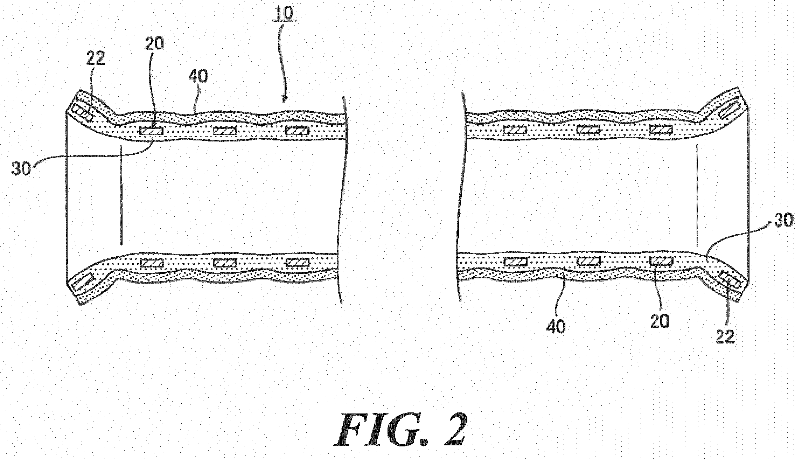

In the embodiment, the inner cover 30 which is made of a polyurethane film covers the stent main body 20 so as to embed the whole of the stent main body 20, such that the mesh-like openings 21 are closed and the end portions 22, 22 are covered (see Figs. 1B and 2). Alternatively, at least one end portion 22 of the stent main body 20 may not be covered by the inner cover 30 to improve the fixing force with respect to a tubular organ such as the bile duct,.

As the polyurethane forming the inner cover 30, for example, polyether type polyurethane, polyester type polyurethane, polycarbonate type polyurethane, polycaprolactone type polyurethane, or the like may be preferably used.

The inner cover 30 can be formed by a method such as dipping, casting, coating, extrusion molding, or spraying. In the case of embedding the stent main body 20 in the inner cover 30 as in the embodiment, dipping or coating is preferably used. Alternatively, the inner cover 30 may be formed by inserting a tube of the above-described material into the inner circumference of the stent main body 20, and by making it adhere to the stent main body 20. Alternatively, the inner cover 30 may be formed by covering the outer circumference of the stent main body 20 with a tube of the above-described material, and by making it diametrically contract and adhere to the stent main body 20 by means such as drying of a solvent or thermal shrinkage.

Preferably, the film thickness of the inner cover 30 is 10 to 60 µm, and more preferably 20 to 40 µm. When the film thickness of the inner cover 30 is smaller than 10 µm, it is difficult to form thinly and uniformly the outer cover 40 which is made of a silicone film. When the film thickness exceeds 60 µm, the whole stent 10 is diametrically enlarged.

On the other hand, the outer circumference of the stent main body 20 and the inner cover 30 is covered by the outer cover 40 which is made of a silicone film. In the embodiment, the outer cover is formed so as to cover the whole stent main body 20 including the flare-like end portions 22, 22, and the whole inner cover 30. When silicone is applied to the outer circumference of the stent, however, the stent becomes slippery, and there is a case where the positional misalignment may occur. In view of this, to at least the one end portion 22 of the stent main body 20, only the inner cover 30 may be disposed while the outer cover 40 may not be disposed, or both the inner cover 30 and the outer cover 40 may not be disposed.

As the silicone forming the outer cover 40, for example, a silicone resin, silicone rubber, a silicone elastomer, or the like can be preferably used.

Similarly with the inner cover 30, for example, the outer cover 40 can be formed on the outer circumference of the stent main body 20 and the inner cover 30 by dipping, casting, coating, extrusion molding, or spraying. Alternatively, the outer cover 40 may be formed by covering the outer circumference of the stent main body 20 and the inner cover 30 with a tube of the above-described material, and by making it diametrically contract by means such as drying of a solvent or thermal shrinkage to make it adhere.

In the embodiment, the whole stent main body 20 is embedded in the inner cover 30, and therefore the whole outer circumference of the inner cover 30 is covered by the outer cover 40.

Preferably, the film thickness of the outer cover 40 is 5 to 60 µm, and more preferably 10 to 40 µm. When the film thickness of the outer cover 40 is smaller than 5 µm, it is difficult to suppress hydrolysis of the inner cover 30 which is made of a polyurethane film. When the film thickness exceeds 60 µm, the whole stent 10 is diametrically enlarged.

Preferably, the total of the film thicknesses of the inner cover 30 and the outer cover 40 is 15 to 120 µm, and more preferably 20 to 100 µm.

Preferably, the above-described stent 10 is configured to have the outer diameter in the contracted state is 1.5 to 2.5 mm, and more preferably 1.6 to 2.4 mm. When the diameter in the contracted state is smaller than 1.5 mm, the stent 10 is too thin such that it is easy to break and the inner cover 30 and the outer cover 40 are tearable. When the diameter exceeds 2.5 mm, the outer diameter of the stent 10 is too large, and it is not preferable.

The inner cover 30 and the outer cover 40 cover the whole stent main body 20, i.e., the range extending to the flared end portions 22, 22. Alternatively, one end portion 22 or both end portions 22, 22 of the stent main body 20 may not be covered. Alternatively, a part of the stent main body 20 may be partially exposed.

A resin such as a silicone resin, a polyurethane resin, a nylon resin, a nylon elastomer, an olefin elastomer such as polybutadiene, or a styrene elastomer, or an interposing layer which is configured by winding a flat membrane formed by these resins may be placed between the inner cover 30 and the outer cover 40.

Next, an example of a method of using the above-described stent 10 will be described.

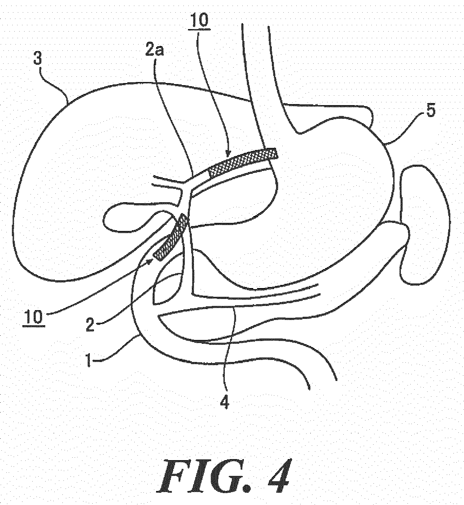

As shown in Fig. 4, the bile duct 2 and the pancreatic duct 4 branch and extend from a lower portion of the duodenum 1 through the papilla, and the bile which is produced by the liver 3 is supplied to the bile duct 2 (common bile duct) through the intrahepatic bile duct 2a.

In the embodiment, as shown in Fig. 4, the stent 10 is to be indwelled between the duodenum I and the bile duct 2 to form an internal fistula to thereby supply the bile the duodenum I bypassing the papilla, and the stent 10 is to be indwelled between the stomach 5 and the intrahepatic bile duct 2a to form an internal fistula to thereby supply the bile to the stomach 5. The procedure of indwelling the stent 10 therefor will be described. First, the former procedure of indwelling the stent 10 between the duodenum 1 and the bile duct 2 will be described.

The stent 10 of the invention is not only used as an internal fistula in which predetermined tubular organs or body tissues are connected to each other to allow a body fluid to flow, but also indwelled in a tubular organ such as the bile duct 2, the pancreatic duct 4, the trachea, the esophagus, the large intestine, or the blood vessel, or another body tissue, The application site is not particularly limited.

First, the stent 10 is contracted, and is accommodated in the inner circumference of a distal end portion of a catheter which is not shown. Thereafter, the duodenum 1 is punctured with a hollow puncture needle by way of the endoscopic ultrasound, and the bile duct 2 is punctured. A guide wire is inserted through the puncture needle, and after the guide wire is extended on the duodenum 1 and the bile duct 2, the puncture needle is pulled out. And, using different diameters of tubes or dilators, an operation of pushing and pulling a tube or dilator through the guide wire is started with a smallest-diameter one and repeated while replacing it with a larger-diameter one. Consequently, the diameters of the puncture holes formed in the duodenum 1 and the bile duct 2 are gradually increased, through several steps.

When the puncture holes are then expanded to a size which allows the catheter accommodating the stent 10 to be passed therethrough, the catheter is inserted through the guide wire by way of the endoscopic ultrasound, and the distal end portion of the catheter is indwelled in the bile duct 2. Then, the stent 10 is released from the distal end of the catheter through a pusher or the like, whereby the stent 10 is indwelled between the duodenum 1 and the bile duct 2. As a result, an internal fistula for supplying the bile into the duodenum 1 bypassing the papilla can be produced.

On the other hand, when the stent 10 is to be indwelled between the stomach 5 and the intrahepatic bile duct 2a, a procedure which is similar to that described above is employed. Namely, the stomach 5 is punctured with a hollow puncture needle by way of the endoscopic ultrasound, and the intrahepatic bile duct 2a is punctured. A guide wire is extended on the stomach 5 and the intrahepatic bile duct 2a, and different diameters of tubes or dilators are sequentially inserted through the guide wire, whereby the diameters of the puncture holes are gradually increased. Thereafter, the catheter accommodating the stent 10 is inserted through the guide wire, the distal end portion of the catheter is indwelled in the intrahepatic bile duct 2a, and then the stent 10 is released from the distal end of the catheter through a pusher or the like. Consequently, the stent 10 is indwelled between the stomach 5 and the intrahepatic bile duct 2a, and an internal fistula for supplying the bile into the stomach 5 can be produced.

According to the stent 10, since the outer circumference of the inner cover 30 which is made of a polyurethane film is covered by the outer cover 40 which is made of a silicone film having high affinity for polyurethane, the silicone film, in which pinholes are easily formed and the thickness is hardly reduced, can be formed thinly and uniformly, and the stent 10 can be diametrically miniaturized. Therefore, the stent 10 can be accommodated even in a catheter of a small inner diameter. Consequently, the number of the above-described operations for increasing the diameter of a puncture hole using tubes or dilators can be reduced, and it is possible to prevent the bile from leaking from the bile duct 2 or the intrahepatic bile duct 2a, thereby suppressing the invasion to the body.

The stent 10 is configured to have an outer diameter in a contracted state of 1.5 to 2.5 mm, and therefore the outer diameter of the stent 10 in the contracted state can be made smaller, so that the stent can be accommodated with margin even in a catheter of a small inner diameter, and invasion to the body can be further suppressed. As described above, the stent 10 can be used for forming an internal fistula between the bile duct 2 having a small inner diameter and the duodenum 1, between the intrahepatic bile duct 2a also having a small inner diameter and the stomach 5, and the like. The stent can be preferably used as a bypass tube for connecting predetermined organs within the body cavity while bypassing a specific organ.

The film thickness of the inner cover 30 is 10 to 60 µm, that of the outer cover 40 is 5 to 60 µm, and the total of the film thicknesses of the covers is 15 to 120 µm. Therefore, the film thicknesses of the inner cover 30 and the outer cover 40 can be reduced within a range where pinholes are not formed, and it is possible to provide the stent 10 which is easily contractable in diameter and has good usability.

By the outer cover 40 made of a silicone film which is hardly hydrolyzed, the polyurethane film of the inner cover 30 can be prevented from being hydrolyzed. Therefore, the stent 10 can be indwelled for a long time period. In a tubular organ in the digestive system, such as the bile duct 2, particularly, the stent 10 can be indwelled for a long time period.

Since the inner cover 30 is made of a polyurethane film, furthermore, irregularities of the inner circumference of the inner cover can be reduced as shown in Fig. 2, and the inner circumference can be made relatively smooth. When the stent 10 is indwelled as an internal fistula between the duodenum 1 and the bile duct 2, or that between the stomach 5 and the intrahepatic bile duct 2a as described above, or when the stent 10 is indwelled in the bile duct 2, therefore, it is possible to suppress sludge such as biliary sludge from being produced inside the stent 10. As a result, it is possible to suppress clogging of the interior of the stent 10, and the stent 10 can be indwelled for a long time period.

### Examples

With respect to the stent of the invention, a degree of reduction of the film thickness of the outer cover that is made of a silicone film is tested.

### (Preparation of examples)

Three examples having the same structure as that of the embodiment shown in Figs. 1A to 3B are produced to the extent that pinholes are not formed in the silicone film of the outer cover.

### (Preparation of comparative examples)

Three comparative examples are also produced to the extent that pinholes are not formed in the silicone film of the outer cover. The three comparative examples have the same structure as that of the embodiment except that the inner cover which is made of a polyurethane film is not provided.

### (Test method)

In each of the examples, the film thickness of the inner cover, that of the outer cover, and the total film thickness of the covers are measured. In each of the comparative examples, the film thickness of the outer cover are measured. Table 1 below shows the measurement results.

**[Table 1]**

| | Layer structure | Film thickness (µm) |
|---|---|---|
| Examples | Inner cover (polyurethane film) | 20 to 40 |
| | Outer cover (silicone film) | 10 to 40 |
| | Inner cover + outer cover | 30 to 80 |
| Comparative examples | Outer cover (silicone film) | 70 to 150 |

### (Test results)

The film thicknesses of the outer covers in the comparative examples are 70 to 150 µm. On the other hand, those of the outer covers in the examples are 10 to 40 µm. The result shows that the silicone film can be formed thinly. Moreover, the film thicknesses of the inner covers in the examples were 20 to 40 µm, and the total film thicknesses of both the covers were 30 to 80 µm. The result also shows that the stent can be diametrically miniaturized.

### Reference Signs List

- 10: stent
- 20: stent main body
- 21: opening
- 30: inner cover
- 40: outer cover

## Claims

1. A stent including:
a tubular stent main body which is formed by processing a metal tube or a metal plate, or by braiding a metal wire material, and which has mesh-like openings;
an inner cover which is made of a polyurethane film, and which covers the stent main body so as to close the mesh-like openings; and
an outer cover which is made of a silicone film, and which covers an outer circumference of the stent main body and the inner cover.

2. The stent of Claim 1,
wherein a film thickness of the inner cover is 10 to 60 µm, a film thickness of the outer cover is 5 to 60 µm, and a total film thickness of these covers is 15 to 120 µm.

3. The stent of Claim 1,
wherein the stent is configured to have an outer diameter in a contracted state of 1.5 to 2.5 mm.
